# EUROPEAN PATENT APPLICATION

(11) **EP 4 710 970 A2**
(43) Date of publication of application: **18.03.2026**
(21) Application number: 25226367.8
(22) Date of filing: 18.11.2021
(51) Int. Cl.: A61M 5/315

(54) **DRUG DELIVERY DEVICE WITH CONTAINER HOLDER**

(30) Priority: 19.11.2020 US 202063116024 P
(62) Divisional of application: 21895560.7
(71) Applicant: Becton, Dickinson and Company, Franklin Lakes, NJ 07417 (US)
(72) Inventor: PALA, Trivikrama, Bhanoji, Parsippany, New Jersey, 07054 (US)
(74) Representative: dompatent

(57) **Abstract**

A drug delivery device includes a housing, a needle having a retracted position and an extended position, a needle actuator body received within the housing and configured to move from a pre-use position where the needle is in the retracted position to a use position where the needle is in the extended position, a container received within the housing, with the container having a first position and a second position spaced axially from the first position, and an actuator button moveable relative to the housing from a first position to a second position to actuate the needle actuator body from the pre-use position to the use position. The actuator button includes a container holder configured to restrict movement of the container from the first position to the second position when the needle actuator body is in the pre-use position.

## Description

### CROSS-REFERENCE TO RELATED APPLICATION

The present application claims priority to United States Provisional Application Serial No. 63/116,024, filed November 19, 2020, entitled "Drug Delivery Device with Container Holder", the entire disclosure of which is hereby incorporated by reference in its entirety.

### BACKGROUND OF THE INVENTION

### Field of the Disclosure

The present disclosure relates to a drug delivery device.

### Description of the Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. When the volume of fluid or drug to be administered is generally below a certain volume, such as 1 mL, an auto-injector is typically used, which typically has an injection time of about 10 to 15 seconds. When the volume of fluid or drug to be administered is above 1 mL, the injection time generally becomes longer resulting in difficulties for the patient to maintain contact between the device and the target area of the patient's skin. Further, as the volume of drug to be administered becomes larger, increasing the time period for injection becomes desirable. The traditional method for a drug to be injected slowly into a patient is to initiate an IV and inject the drug into the patient's body slowly. Such a procedure is typically performed in a hospital or outpatient setting.

Certain devices allow for self-injection in a home setting and are capable of gradually injecting a liquid therapeutic preparation into the skin of a patient. In some cases, these devices are small enough (both in height and in overall size) to allow them to be "worn" by a patient while the liquid therapeutic preparation is being infused into the patient. These devices typically include a pump or other type of discharge mechanism to force the liquid therapeutic preparation to flow out of a reservoir and into the injection needle. Such devices also typically include a valve or flow control mechanism to cause the liquid therapeutic preparation to begin to flow at the proper time and a triggering mechanism to initiate the injection.

### SUMMARY OF THE INVENTION

In one aspect or embodiment, a drug delivery device includes a housing, a needle having a retracted position and an extended position, a needle actuator body received within the housing and configured to move from a pre-use position where the needle is in the retracted position to a use position where the needle is in the extended position, a container received within the housing, and an actuator button moveable relative to the housing from a first position to a second position to actuate the needle actuator body from the pre-use position to the use position. The container includes a body, a closure, and a stopper moveable within the body, with the container having a first position and a second position spaced axially from the first position. The actuator button includes a container holder configured to restrict movement of the container from the first position to the second position when the needle actuator body is in the pre-use position.

The container holder may be a projection configured to engage the body of the container. The body of the container may include a flange, with the container holder configured to engage with the flange of the container.

The device may further include a valve assembly including a valve boot and a valve needle, where the container is spaced from the valve assembly when the container is in the first position, and where the container is engaged with the valve assembly when the container is in the second position.

The device may further include a drive assembly configured to move the container from the first position to the second position and configured to move the stopper relative to the body of the container. The actuator button may be configured to actuate the drive assembly.

The needle actuator body may include a projection configured to restrict movement of the container from the first position to the second position when the needle actuator body is in the pre-use position. The projection of the needle actuator body may be spaced from the container holder of the actuator button in a direction extending perpendicular to a longitudinal axis of the housing.

The actuator button may be spaced from the container when the container is in the second position. The container holder may be formed integrally with the actuator button.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIG. 1 is a perspective view of a drug delivery device according to one aspect or embodiment of the present application.
FIG. 2 is a perspective, cross-sectional view of the drug delivery device of FIG. 1.
FIG. 3 is a front, cross-sectional view of the drug delivery device of FIG. 1.
FIG. 4 is a top view of the drug delivery device of FIG. 1, showing a top portion of the housing removed and the drug delivery device in a pre-use position.
FIG. 5 is a top, cross-sectional view of the drug delivery device of FIG. 1, showing the drug delivery device in a pre-use position.
FIG. 6 is a front, cross-sectional view of the drug delivery device of FIG. 1, showing the drug delivery device in a pre-use position.
FIG. 7 is a top view of the drug delivery device of FIG. 1, showing a top portion of the housing removed and the drug delivery device in an initial actuation position.
FIG. 8 is a top, cross-sectional view of the drug delivery device of FIG. 1, showing the drug delivery device in an initial actuation position.
FIG. 9 is a front, cross-sectional view of the drug delivery device of FIG. 1, showing the drug delivery device in an initial actuation position.
FIG. 10 is a top view of the drug delivery device of FIG. 1, showing a top portion of the housing removed and the drug delivery device in a use position.
FIG. 11 is a top, cross-sectional view of the drug delivery device of FIG. 1, showing the drug delivery device in a use position.
FIG. 12 is a front, cross-sectional view of the drug delivery device of FIG. 1, showing the drug delivery device in a use position.
FIG. 13 is a top view of the drug delivery device of FIG. 1, showing a top portion of the housing removed and the drug delivery device in a post-use position.
FIG. 14 is a top, cross-sectional view of the drug delivery device of FIG. 1, showing the drug delivery device in a post-use position.
FIG. 15A is a front, cross-sectional view of the drug delivery device of FIG. 1, showing the drug delivery device in a post-use position.
FIG. 15B is a front, cross-sectional view of the drug delivery device of FIG. 1, showing the drug delivery device in a pre-use position.
FIG. 15C is a perspective, cross-sectional view of the drug delivery device of FIG. 1, showing the drug delivery device in a pre-use position.
FIG. 16 is a partial perspective view of the drug delivery device of FIG. 1, showing a top portion of the housing removed and the drug delivery device in a pre-use position.
FIG. 17 is partial perspective view of the drug delivery device of FIG. 1, showing a top portion of the housing removed and the drug delivery device in a pre-use position.

Corresponding reference characters indicate corresponding parts throughout the several views. The exemplifications set out herein illustrate exemplary aspects of the disclosure, and such exemplifications are not to be construed as limiting the scope of the disclosure in any manner.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

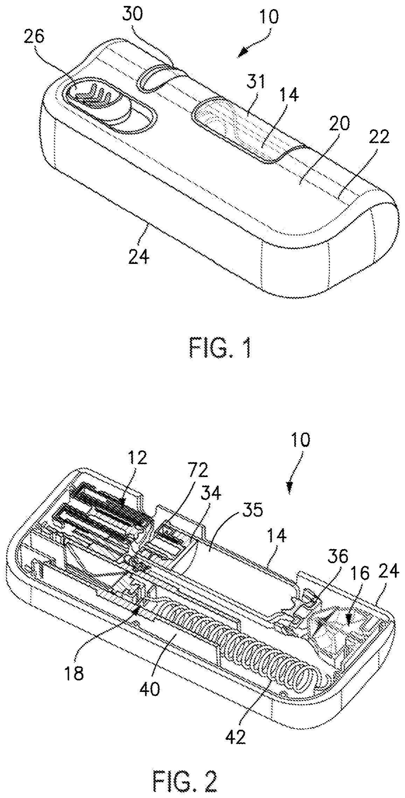

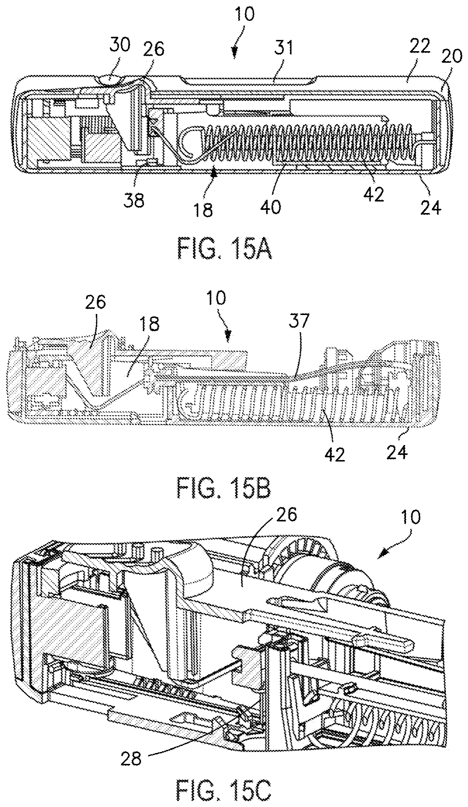

Referring to FIGS. 1-17, a drug delivery device 10 includes a drive assembly 12, a container 14, a valve assembly 16, and a needle actuator assembly 18. The drive assembly 12, the container 14, the valve assembly 16, and the needle actuator assembly 18 are at least partially positioned within a housing 20. The housing 20 includes a top portion 22 and a bottom portion 24, although other suitable arrangements for the housing 20 may be utilized. In one aspect, the drug delivery device 10 is an injector device configured to be worn or secured to a person and to deliver a predetermined dose of a medicament provided within the container 14 via injection into the person. The device 10 may be utilized to deliver a "bolus injection" where a medicament is delivered within a set time period. The medicament may be delivered over a time period of up to 45 minutes, although other suitable injection amounts and durations may be utilized. A bolus administration or delivery can be carried out with rate controlling or have no specific rate controlling. The device 10 may deliver the medicament at a fixed pressure to the user with the rate being variable. The general operation of the device 10 is described below in reference to FIGS. 1-17.

Referring again to FIGS. 1-17, the device 10 is configured to operate through the engagement of an actuation button 26 by a user, which results in a needle 28 of the needle actuator assembly 18 piercing the skin of a user, the actuation of the drive assembly 12 to place the needle 28 in fluid communication with the container 14 and to expel fluid or medicament from the container 14, and the withdrawal of the needle 28 after injection of the medicament is complete. The operation of the device 10 may operate in the same manner as shown and described in U.S. Publication No. 2017/0354788, which is hereby incorporated by reference in its entirety. The housing 20 of the device 10 includes an indicator window 30 for viewing an indicator arrangement 32 configured to provide an indication to a user on the status of the device 10 and a container window 31 for viewing the container 14. The indicator window 30 may be a magnifying lens for providing a clear view of the indicator arrangement 32. The indicator arrangement 32 moves along with the needle actuator assembly 18 during use of the device 10 to indicate a pre-use status, use status, and post-use status of the device 10. The indicator arrangement 32 provides visual indicia regarding the status, although other suitable indicia, such an auditory or tactile, may be provided as an alternative or additional indicia.

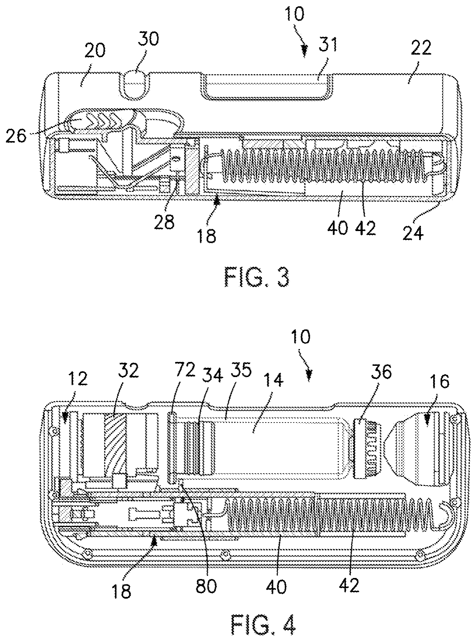

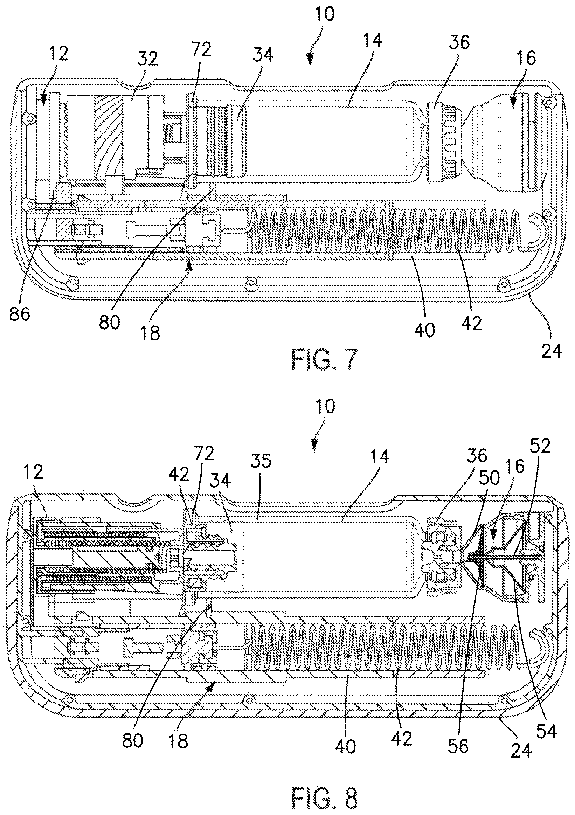

Referring to FIGS. 4-6, during a pre-use position of the device 10, the container 14 is spaced from the drive assembly 12 and the valve assembly 16 and the needle 28 is in a retracted position. During the initial actuation of the device 10, as shown in FIGS. 7-9, the drive assembly 12 is released after movement of the actuator button 26 and engages the container 14 to move the container 14 from a first position to a second position spaced axially from the first position with the container 14 engaged with the valve assembly 16. Moving the container 14 toward the valve assembly 16 is configured to pierce a closure 36 of the container 14 and place the medicament within the container 14 in fluid communication with the needle 28 via a tube 37 or other suitable arrangement. The drive assembly 12 is configured to engage a stopper 34 positioned within a body or barrel 35 of the container 14, which will initially move the entire container 14 into engagement with the valve assembly 16 due to the incompressibility of the fluid or medicament within the container 14. The initial actuation of the device 10 is caused by engagement of the actuation button 26 by a user, which releases the needle actuator assembly 18 and the drive assembly 12 as discussed below in more detail. During the initial actuation, the needle 28 is still in the retracted position and about to move to the extended position to inject the user of the device 10.

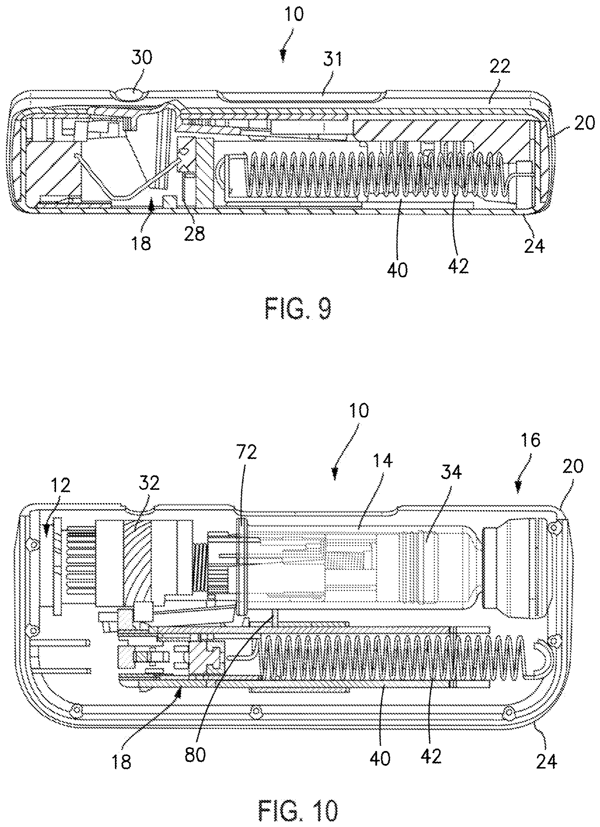

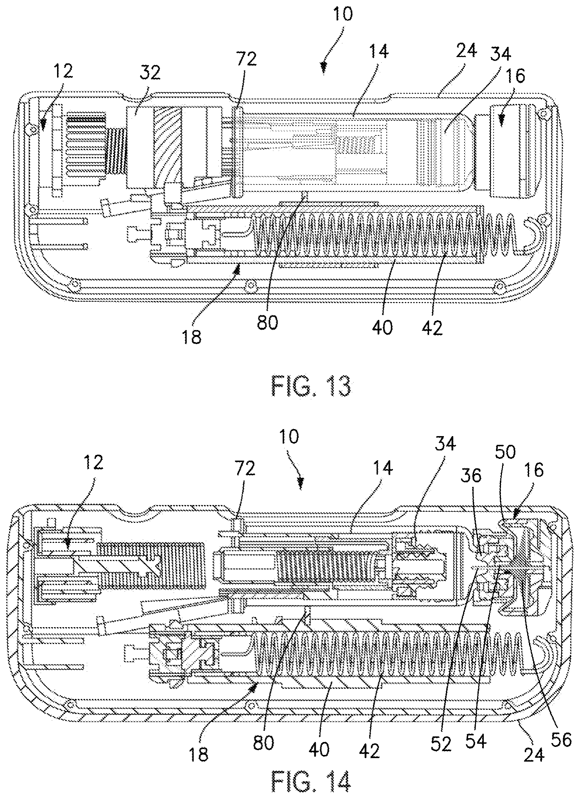

During the use position of the device 10, as shown in FIGS. 10-12, the needle 28 is in the extended position at least partially outside of the housing 20 with the drive assembly 12 moving the stopper 34 within the container 14 to deliver the medicament from the container 14, through the needle 28, and to the user. In the use position, the valve assembly 16 has already pierced a closure 36 of the container 14 to place the container 14 in fluid communication with the needle 28, which also allows the drive assembly 12 to move the stopper 34 relative to the container 14 since fluid is able to be dispensed from the container 14. At the post-use position of the device 10, shown in FIGS. 13-15A, the needle 28 is in the retracted position and engaged with a pad 38 to seal the needle 28 and prevent any residual flow of fluid or medicament from the container 14.

Referring again to FIGS. 1-17, the needle actuator assembly 18 includes a needle actuator body 40 received within the housing 20 and configured to move from a pre-use position (FIGS. 3-6) where the needle 28 is in the retracted position to a use position (FIGS. 10-12) where the needle 28 is in the extended position. The needle 28 is entirely positioned within the housing 20 in the retracted position and at least partially positioned outside of the housing 20 in the extended position. The needle actuator body 40 also has a post-use position (FIGS. 13-15A) where the needle 28 is in the retracted position and engaged with the pad 38, as discussed above. The needle actuator body 40 is biased from the pre-use position to the post-use position by a spring 42. The actuator button 26, as discussed above, is moveable relative to the housing 20 from a first position to a second position to actuate the needle actuator body 40 from the pre-use position to the use position. In one aspect or embodiment, movement of the actuator button 26 from the first position to the second position moves the needle actuator body 40 to release the needle actuator body 40 from engagement with the housing 20 or another component to allow the spring 42 to bias the needle actuator body 40 from the pre-use position, to the use position, and to the post-use position. The needle actuator body 40 moves the needle 28 between the retracted and extended positions via a camming arrangement as the needle actuator body 40 moves between the positions, although other suitable arrangements may be utilized.

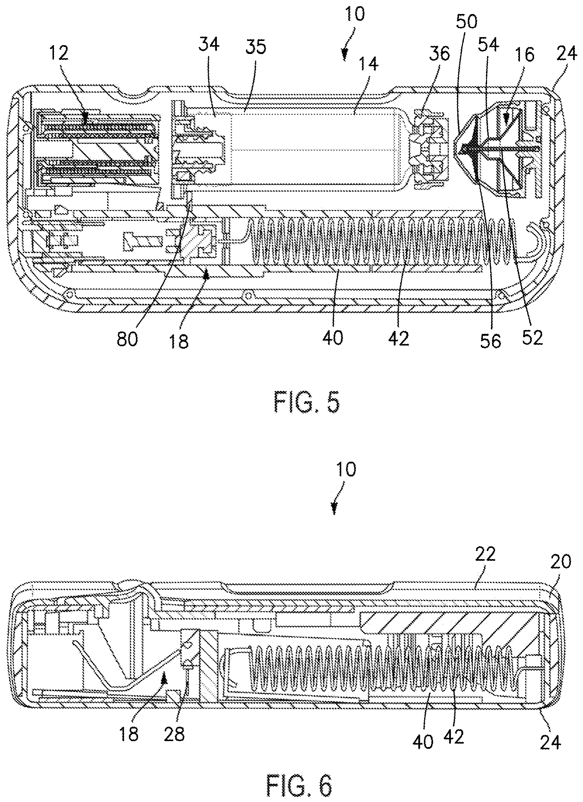

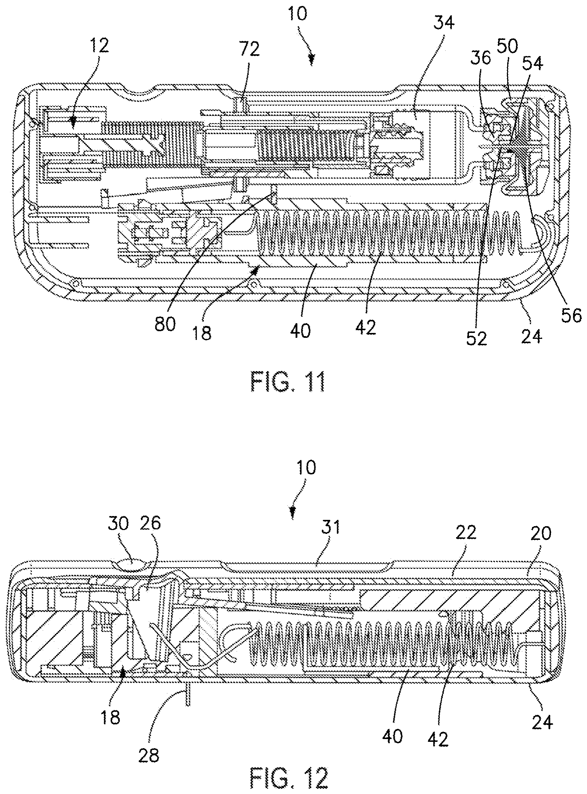

Referring to FIGS. 5, 8, and 11, in one aspect or embodiment, the valve assembly 16 includes a valve boot 50, a valve needle 52, a valve sleeve 54, and a pierce plate 56, although other suitable valve arrangements may be utilized. During use, the closure 36 of the container 14 initially contacts the valve boot 50 (FIG. 8), which compresses the valve boot 50 until the pierce plate 56 pierces the valve boot 50. As the container 14 moves to the position shown in FIG. 11, the valve boot 50 fully compresses, the valve sleeve 54 compresses, and the valve needle 52 extends through the closure 36 of the container 14 to place the contents of the container 14 in fluid communication with the valve needle 52. As noted above, the valve needle 52 is in fluid communication with the needle 28 via the tube 37. In one aspect or embodiment, the closure 36 of the container 14 includes a foil seal, a septum, and a locking collar, although other suitable arrangements may be utilized.

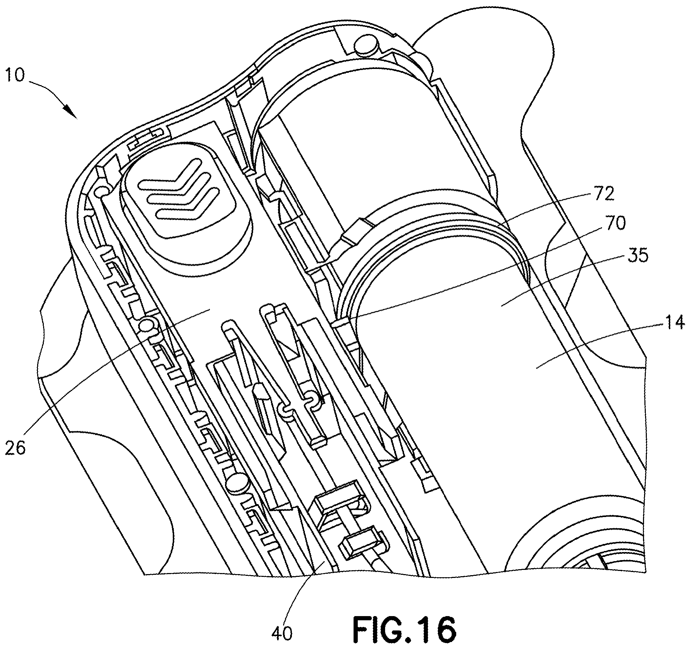

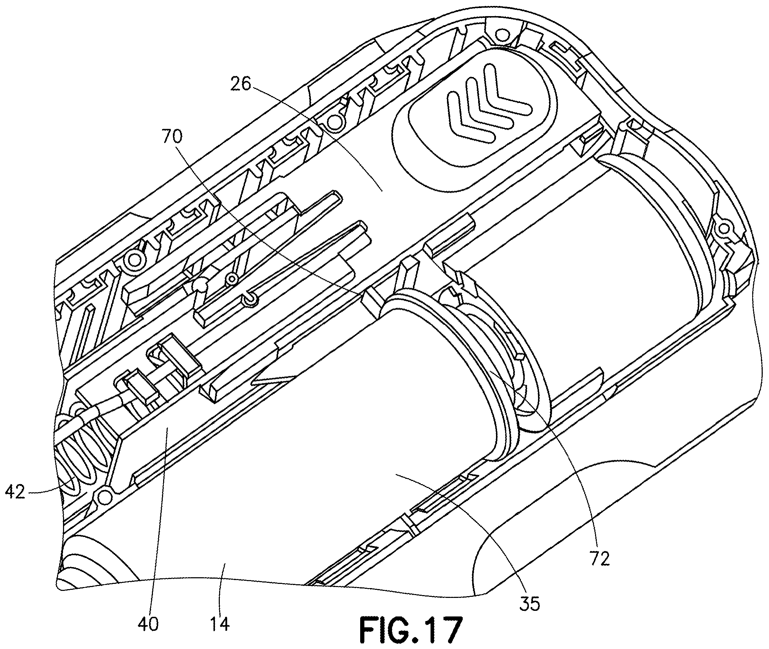

Referring to FIGS. 16 and 17, the actuator button 26 includes a container holder 70 configured to restrict movement of the container 14 from the first position to the second position when the needle actuator body 40 is in the pre-use position. In one aspect or embodiment, the container holder 70 is configured to prevent the container 14 from moving into engagement with the valve assembly 16 prior to actuation of the device 10 if the device 10 is dropped or exposed to sudden acceleration or deceleration. Contact between the container 14 and the valve assembly 16 may cause the valve boot 50 or other portion of the valve assembly 16 to be pierced or a portion of the closure 36 of the container 14 to be pierced, which may compromise the sterility of the container 14 or the valve assembly 16. The container holder 70 is a rectangular projection configured to engage the body 35 of the container 14. In certain configurations, the projection may be a square, a cylinder, or any other suitable shape. In one aspect or embodiment, the container holder 70 engages a flange 72 of the container 14. In one aspect or embodiment, the container holder 70 is formed integrally with the actuator button 26, although the container holder 70 may be formed separately from the actuator button 26.

When the needle actuator body 40 is in the pre-use position and prior to actuation of the device 10, the container holder 70 restricts the movement of the container 14 if the device 10 is dropped. In one aspect or embodiment the container holder 70 is spaced from the flange 72 of the container 14 a predetermined distance that avoids interfering with the container 14 after actuation of the device 10 while stopping movement of the container 14 prior to engagement of the container 14 with the valve assembly 16. There should exist a clearance between container holder 70 and the outer perimeter of the container 14 such that the container holder 70 can absorb the load of the barrel movement. Accordingly, the actuator button 26 is spaced from the container 14 during use of the device 10.

Referring to FIGS. 4 and 5, the needle actuator body 40 includes a projection 80 configured to restrict movement of the container 14 from the first position to the second position when the needle actuator body 40 is in the pre-use position. The projection 80 of the needle actuator body 40 is spaced from the container holder 70 of the actuator button 26 in a direction extending perpendicular to a longitudinal axis of the housing 20. In one aspect or embodiment, the projection 80 is configured to contact a bottom portion of the flange 72 of the container 14 while the container holder 70 is configured to contact a top portion of the flange 72 of the container 14. Providing two points of contact with the container 14 ensures the container 14 does not contact the valve assembly 16 prior to actuation of the device 10 for all potential orientations that the device 10 may be dropped. Only contacting a bottom portion of the flange 72 may allow rocking or other movement of the container 14 for certain drop orientations of the device 10, which may allow undesirable contact between the container 14 and the valve assembly, as discussed above.

Elements of one disclosed aspect can be combined with elements of one or more other disclosed aspects to form different combinations, all of which are considered to be within the scope of the present invention.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A drug delivery device comprising:
a housing;
a needle having a retracted position and an extended position;
a needle actuator body received within the housing and configured to move from a pre-use position where the needle is in the retracted position to a use position where the needle is in the extended position;
a container received within the housing, the container comprising a body, a closure, and a stopper moveable within the body, the container having a first position and a second position spaced axially from the first position; and
an actuator button moveable relative to the housing from a first position to a second position to actuate the needle actuator body from the pre-use position to the use position, the actuator button comprising a container holder configured to restrict movement of the container from the first position to the second position when the needle actuator body is in the pre-use position.

2. The drug delivery device of claim 1, wherein the container holder comprises a projection configured to engage the body of the container.

3. The drug delivery device of claim 2, wherein the body of the container comprises a flange, the container holder configured to engage with the flange of the container.

4. The drug delivery device of any of claims 1-3, further comprising a valve assembly comprising a valve boot and a valve needle, wherein the container is spaced from the valve assembly when the container is in the first position, and wherein the container is engaged with the valve assembly when the container is in the second position.

5. The drug delivery device of any of claims 1-4, further comprising a drive assembly configured to move the container from the first position to the second position and configured to move the stopper relative to the body of the container.

6. The drug delivery device of claim 5, wherein the actuator button is configured to actuate the drive assembly.

7. The drug delivery device of any of claims 1-6, wherein the needle actuator body comprises a projection configured to restrict movement of the container from the first position to the second position when the needle actuator body is in the pre-use position.

8. The drug delivery device of claim 7, wherein the projection of the needle actuator body is spaced from the container holder of the actuator button in a direction extending perpendicular to a longitudinal axis of the housing.

9. The drug delivery device of any of claims 1-8, wherein the actuator button is spaced from the container when the container is in the second position.

10. The drug delivery device of any of claims 1-9, wherein the container holder is formed integrally with the actuator button.
